# EUROPÄISCHE PATENTSCHRIFT

(11) **EP 0 723 949 B1**
(45) Veröffentlichungstag und Bekanntmachung des Hinweises auf die Patenterteilung: **26.04.2000**
(21) Anmeldenummer: 96100680.6
(22) Anmeldetag: 18.01.1996
(51) Int. Cl.: C07C 45/36, C07C 47/55

(54) **Verfahren zur Herstellung von Halogenbenzaldehyden**
Process for the preparation of halogenobenzaldehydes
Procédé pour la préparation de benzaldéhydes halogénés

(30) Priorität: 30.01.1995 DE 19502805
(43) Veröffentlichungstag der Anmeldung: 31.07.1996
(73) Patentinhaber: Aventis Research & Technologies GmbH & Co. KG, 65926 Frankfurt am Main (DE)
(72) Erfinder: Borchert, Holger, Dr., D-65929 Frankfurt (DE); Gerdau, Thomas, Dr., D-65817 Eppstein (DE); Weiguny, Jens, Dr., D-64331 Weiterstadt (DE)

(56) Entgegenhaltungen:
- EP-A- 0 226 640
- DE-B- 1 202 774
- CHEMICAL ABSTRACTS, vol. 080, no. 21, 27.Mai 1974 Columbus, Ohio, US; abstract no. 119931, CHOPRA B ET AL: "Oxidation of chlorotoluenes on bismuth oxide-molybdenum oxide" XP002002216 & INDIAN CHEM. J., ANNU. (ICHJAU);72; PP.38-49, INDIAN INST. TECHNOL.;CHEM. DEP.; DELHI; INDIA,

## Beschreibung

Die Erfindung betrifft ein Verfahren zur Herstellung von Halogenbenzaldehyden durch katalytische Gasphasenoxidation eines substituierten Toluols mit Sauerstoff.

Zur Herstellung von Halogenbenzaldehyden ist die Seitenkettenchlorierung der Toluole mit anschließender Hydrolyse der entstehenden Benzalchloride bekannt (DE 4 239 736). Dieses Verfahren ist durch hohe Chlorkosten sowie aufwendige Abwasserbeseitigung nicht ökonomisch vorteilhaft. Als weitere Techniken zur Herstellung von Benzaldehyden, die in einer der ortho- oder para-Stellungen einen elektronenanziehenden Substituenten tragen, sind die Flüssigphasenoxidation von substituierten β-Aminostyrolen in Gegenwart von Kupfersalzen (EP 0 430 001) und die Flüssigphasenoxidation von Halogentoluolen in Gegenwart von Kobalt- und Mangansalzen (JP 54 100 336, JP 54 109 938) beschrieben. Die Nachteile dieser Verfahren sind neben ihrer außerordentlichen Kompliziertheit die Notwendigkeit zur Aufarbeitung der Lösungsmittel und Regenerierung der Metallsalze.

Für die Herstellung von Benzaldehyd durch Gasphasenoxidation von Toluol mit molekularem Sauerstoff sind als Katalysatoren Silbervanadat entweder allein oder in Kombination mit Blei- und Eisenoxid (SU 495 301, DE 1 295 538, NL 7 607 598) sowie ein Mischoxidkatalysator bestehend aus Kupfer-, Eisen-, Uran, Blei-, Tellur-, Molybdän- und Phosphoroxid (US 4 390 728) bekannt. Mit diesem Katalysator wird bei 38 % Umsatz Benzaldehyd mit 64 % Selektivität gebildet.

Höhere Ausbeuten werden bei der Oxidation von methoxy-, phenoxy- oder tert.-butylsubstituierten Toluolen erhalten, was auf den positiven Induktionseffekt dieser Gruppen zurückgeführt wurde (Grybowska, 1987; Ueshima, 1992; Constantini, 1986). So können p-Methoxytoluol und p-Phenoxytoluol an einem Vanadium-Thallium-Cäsium-Antimon- bzw. Vanadium-Cäsium-Kalium-Phosphor-Eisen-Kobalt-Oxidkatalysator bei 450°C mit über 75 % Ausbeute zu den entsprechenden Aldehyden oxidiert werden (EP 226 640). Am letztgenannten Katalysator wird entsprechend dem geringeren elektronenschiebenden Effekt des tert.-Butylsubstituenten im Vergleich zum Methoxy- und Phenoxysubstituenten p-tert.-Butylbenzaldehyd mit 56 % Ausbeute erhalten (EP 226 640).

Im Gegensatz zu Toluolen mit elektronenschiebenden Substituenten am aromatischen Ring wurden bislang nur geringe Ausbeuten im Falle der Gasphasenoxidation von Halogentoluolen erhalten, was das Vorurteil zu bestätigen scheint, daß hohe Selektivitäten im Falle von elektronenziehenden Substituenten nicht zu erreichen sind. Nach DT 1 202 774 kann 2,6-Dichlortoluol lediglich mit 13 % Ausbeute zum entsprechend substituierten Benzaldehyd an Wismutmolybdat oxidiert werden. Der Umsatz bei einmaligem Reaktordurchgang beträgt nur 20 %, obgleich die Reaktionstemperatur bei 580°C liegt. Bei der Oxidation von p-Chlortoluol in einer Natriumnitrat/Kaliumnitrat-Schmelze beträgt die Ausbeute sogar nur 4 % (US 4 885 412). Die für die p-Chlortoluoloxidation an Oxovanadium(IV)-diphosphat als Katalysator berichteten Ausbeuten von 85 % (DD 298 234) sind, wie Vergleichsversuche zeigten, nicht zu erreichen. Mit diesem Katalysator können lediglich 7 % Ausbeute erzielt werden (siehe Vergleichsbeispiel 2).

Es besteht also ein Bedarf nach einem Verfahren, das die genannten Nachteile vermeidet und es ermöglicht, Halogenbenzaldehyde durch die kostengünstige Direktoxidation der substituierten Toluole mit molekularem Sauerstoff in hoher Selektivität zu erhalten.

Diese Aufgabe wird gelöst durch ein Verfahren zur Herstellung von Halogenbenzaldehyden der allgemeinen Formel (I): 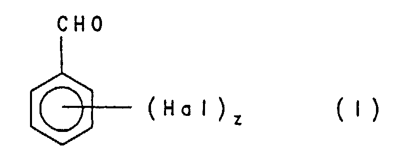 in welcher Hal für Fluor, Chlor, Brom oder Jod steht und z = 1, 2, 3 oder 4 ist, durch katalytische Gasphasenoxidation eines substituierten Toluols der allgemeinen Formel (II), 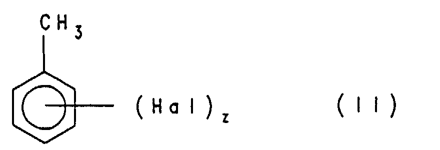 mit Sauerstoff, dadurch gekennzeichnet, daß die Oxidation in Gegenwart eines Katalysators der allgemeinen Formel (III)

Me¹ ₐMe² _{b}Me³ _{c}Oₓ (III)

durchgeführt wird, in der Me¹ für Wismut oder Vanadium, Me² für mindestens eines der Elemente aus der Gruppe bestehend aus Lithium, Natrium, Kalium, Rubidium und Cäsium, Me³ für mindestens ein Element aus der Gruppe bestehend aus Eisen, Kobalt, Nickel, Niob, Molybdän, Arsen, Zinn, Antimon und Rhenium, Wolfram, Tantal, Phosphor, Chrom, Mangan, Palladium, Kupfer, Zink, Cer, Silber, Bor, Samarium, Barium, Calcium, Magnesium stehen, und die Buchstaben a, b und c ein solches Atomverhältnis der jeweiligen Elemente darstellen, daß dann wenn a = 1 angenommen wird, b einen Wert im Bereich von 0,1 bis 2 und c einen Wert im Bereich von 0,05 bis 2 hat, wobei für unterschiedliche Elemente Me² oder Me³ unterschiedliche Werte für b bzw. c möglich sind.

Gute Ergebnisse werden z.B. mit einem Katalysator erzielt, worin Me³ für mindestens ein Element aus der Gruppe Eisen, Kobalt, Nickel, Niob, Molybdän, Arsen, Zinn, Antimon und Rhenium steht.

Wenn Me¹ = Vanadium, erweist sich eine Kombination A, bei der Me² Cäsium und Me³ mindestens ein Element aus der Gruppe bestehend aus Eisen, Niob, Arsen, Zinn, Antimon, Rhenium und Wolfram ist, insbesondere die Kombination V₁Cs_{0,5-1}Fe_{0,5-2}(Nb, Sb, Sn, Re oder W)_{0,05-0,2}Oₓ, als besonders zweckmäßig. Wenn Me¹ = Wismut, erweist sich eine Kombination B, in der Me² Cäsium und Me³ mindestens ein Element aus der Gruppe bestehend aus Molybdän, Zinn, Antimon, Eisen und Kobalt ist, insbesondere die Kombination Bi₁Cs_{0,5-1}Mo_{0,5-2} (Sb und/oder Sn)_{0,2-1,5}Oₓ als besonders zweckmäßig.

Der Katalysator kann in reiner Form vorliegen, mit einem Trägermaterial gemischt sein oder auf einem geformten Trägermaterial fixiert sein. Beispiele für Trägermaterialien sind Aluminiumoxid, Keramik, Kieselgur, Kieselgel, Siliciumcarbid, geglühtes Siliciumdioxid, Titandioxid und dergleichen. Vorzugsweise wird ein Katalysator der Zusammensetzung A auf einem Trägermaterial fixiert. Besonders bevorzugtes Trägermaterial ist Titandioxid. Ein Katalysator der Zusammensetzung B wird vorzugsweise in reiner Form eingesetzt.

Die Ausgangsmaterialien der Einzelkomponenten zur Herstellung des erfindungsgemäßen Katalysators sind neben den Oxiden vorzugsweise in Wasser lösliche Substanzen wie Ammoniumsalze, Nitrate, Sulfate, Halogenide, Hydroxide und Salze organischer Säuren, die durch Erwärmung in die entsprechenden Oxide umgewandelt werden können. Zur Vermischung der Komponenten werden wäßrige Lösungen der Metallsalze hergestellt und vermischt. Der inerte Träger kann in dieser Mischung suspendiert werden. Nach Eindampfen wird der Katalysatorvorläufer bei Temperaturen zwischen 100 und 250°C verformt, anschließend bei 300 bis 1000°C, bevorzugt bei 400 bis 800°C calciniert. Als Zeit für die Kalzination kommen 2 bis 24 Stunden in Frage.

Die Reaktion kann in der Wirbelschicht oder in einem Festbettreaktor durchgeführt werden. Für den Einsatz in eine Wirbelschicht wird der Katalysator auf eine Korngröße im Bereich von 10 µm bis 200 µm gemahlen. Als Oxidationsmittel wird Sauerstoff oder ein sauerstoffhaltiges Gas wie Luft oder eine Mischung von Sauerstoff mit einem inerten Gas wie Stickstoff, Wasserdampf oder Edelgasen verwendet. Bevorzugt ist die Verwendung von Luft. Der Gehalt des Halogentoluols im Ausgangsgemisch kann innerhalb eines breiten Bereichs variieren, wobei natürlich die Selbstentzündungstemperatur und die Explosionsgrenzen des Halogentoluols beachtet werden müssen. Vorteilhafterweise wird das Verfahren ausgeführt, indem der Eduktstrom bestehend aus 0,5 bis 5,0 Vol.-% Halogentoluol und 99,5 bis 95,0 Vol.-% Luft mit einer Raumgeschwindigkeit von 100 bis 1000 h⁻¹ (STP = Standard Temperatur und Druck: 273°K, 10⁵Pa) bei einer Reaktionstemperatur von 400 bis 500°C in den Reaktor eingetragen wird.

Großes technisches Interesse hat das Verfahren, wenn 2-Chlortoluol, 4-Chlortoluol, 4-Fluortoluol oder 2-Bromtoluol eingesetzt werden.

Wichtig ist das Verfahren zur Herstellung von Verbindungen der Formel (I), worin Hal für Fluor, Chlor oder Brom, insbesondere Chlor oder Brom, bevorzugt Chlor steht und z = 1 oder 2, insbesondere 1 ist.

### Beispiele

### Beispiel 1

Es wird eine Lösung aus 10,0 g Ammoniummetavanadat und 14,0 g Oxalsäure in warmen Wasser hergestellt. Zu dieser Lösung werden 34,5 g Eisennitrat und 8,3 g Cäsiumnitrat hinzugegeben. Die Mischung wird für eine Stunde gerührt, anschließend 6,0 g Titandioxid hinzugegeben und für weitere 30 Minuten gerührt. Unter Rühren wird die Suspension eingedampft. Nach dem Trocknen wird der Katalysatorvorläufer bei 500°C für 6 Stunden calciniert. Der Katalysator wird nach Abkühlen auf Raumtemperatur im Mörser homogenisiert und das Pulver zu einer Siebfraktion (18 bis 25 mesh) verarbeitet.

Zur Durchführung der Oxidation wird 20 g des Katalysators mit 20 g Quarzglasbruch derselben Siebfraktion vermischt und diese Mischung in ein Quarzglasrohr mit 1,5 cm Innendurchmesser gegeben. Zur Thermostatisierung taucht der Reaktor in ein fluidisiertes Sandbad ein, das von außen beheizt wird. Das Eduktgas bestehend aus 1 Vol.-% 4-Chlortoluol und 99 Vol.-% Luft wird mit einem Gesamtvolumenstrom von 2,5 ml/s (STP) durch den beheizten Reaktor geleitet und das Produkt in einer bei -70°C gekühlten Falle gesammelt. Mittels Gaschromatographie werden Kohlenmonoxid und Kohlendioxid quantitativ erfaßt. Das in der Kühlfalle gesammelte nicht umgesetzte 4-Chlortoluol sowie der gebildete 4-Chlorbenzaldehyd werden in Acetonitril aufgenommen und mit HPLC analysiert. Die Ergebnisse der Reaktion sind in Tabelle 1 angegeben.

### Beispiel 2

Das in Beispiel 1 beschriebene Verfahren wird durchgeführt mit der Abweichung, daß zur wäßrigen Lösung zusätzlich 2,9 g Nioboxalat gegeben wurde. Die Ergebnisse sind in Tabelle 1 angegeben.

### Beispiel 3

Das in Beispiel 1 beschriebene Verfahren wird durchgeführt mit der Abweichung, daß zur wäßrigen Lösung zusätzlich 2,0 g Zinnchlorid gegeben wurde. Die Ergebnisse sind in Tabelle 1 angegeben.

### Beispiel 4

Es wird eine Lösung aus 10,0 g Ammoniummetavanadat und 14,0 g Oxalsäure in warmen Wasser hergestellt. Zu dieser Lösung werden 8,3 g Cäsiumnitrat und 4,9 g Kaliumperrhenat hinzugegeben. Die Mischung wird für eine Stunde gerührt, anschließend 7,9 g Titandioxid hinzugegeben und für weitere 30 Minuten gerührt. Unter Rühren wird die Suspension eingedampft. Nach dem Trocknen wird der Katalysatorvorläufer bei 500°C für 6 Stunden calciniert.

Die Oxidation wird wie in Beispiel 1 beschrieben durchgeführt mit der Abweichung, daß die Katalysatoreinwaage 40 g betrug. Die Ergebnisse sind in Tabelle 1 angegeben.

### Beispiel 5

Das im Beispiel 1 beschriebene Verfahren wird durchgeführt mit der Abweichung, daß zur wäßrigen Lösung zusätzlich 2,4 g Ammoniumwolframat gegeben wurde.

Die Oxidation wird wie in Beispiel 1 beschrieben durchgeführt mit der Abweichung, daß die Katalysatoreinwaage 10 g betrugt.
Die Ergebnisse sind in Tabelle 1 angegeben.

### Bespiel 6

Zu einer Lösung aus 26,5 g Ammoniumheptamolybdat in 100 ml Wasser wird eine Lösung aus 43,1 g Wismutnitrat in 100 ml 50 %iger Salpetersäure zugetropft, wobei durch Zugabe von Ammoniaklösung ein pH-Wert zwischen 7 und 8 eingestellt wurde. Anschließend werden 14,6 g Cäsiumnitrat, 4,0 g Eisennitrat, 2,9 g Kobaltnitrat, 14,6 g Antimonoxid und 15,1 g Zinnoxid hinzugegeben und die Mischung langsam eingedampft. Nach dem Trocknen wird der Katalysatorvorläufer bei 600°C für 12 Stunden calciniert. Der Katalysator wird nach dem Abkühlen auf Raumtemperatur im Mörser homogenisiert und das Pulver zu einer Siebfraktion (18 bis 25 mesh) verarbeitet.

Die Oxidation wird wie in Beispiel 1 beschrieben durchgeführt mit der Abweichung, daß die Katalysatoreinwaage 10 g betrug. Die Ergebnisse sind in Tabelle 1 angegeben.

### Beispiel 7

Das in Beispiel 5 beschriebene Verfahren wird durchgeführt mit der Abweichung, daß zur wäßrigen Wismutnitrat/Ammoniummolybdatmischung 24,4 g Cäsiumnitrat, 4,0 g Eisennitrat, 2,9 g Kobaltnitrat, 59,3 g Antimonchlorid und 45,1 g Zinnchlorid hinzugegeben wird.

Die Oxidation wird wie in Beispiel 1 beschrieben durchgeführt. Die Ergebnisse sind in Tabelle 1 angegeben.

### Beispiel 8

Das in Beispiel 2 beschriebene Verfahren wird durchgeführt mit der Abweichung, daß statt 4-Chlortoluol 2-Chlortoluol als Halogentoluol eingesetzt wurde. Die Ergebnisse sind in Tabelle 1 angegeben.

### Beispiel 9

Beispiel 6 wird durchgeführt mit der Abweichung, daß statt 4-Chlortoluol 2-Chlortoluol als Halogentoluol eingesetzt wird. Die Ergebnisse sind in Tabelle 1 angegeben.

### Beispiel 10

Das in Beispiel 2 beschriebene Verfahren wird durchgeführt mit der Abweichung, daß statt 4-Chlortoluol 4-Fluortoluol als Halogentoluol eingesetzt wurde.
Die Ergebnisse sind in Tabelle 1 angegeben.

### Vergleichsbeispiel 1

Zu einer Lösung aus 26,5 g Ammoniumheptamolybdat in 100 ml Wasser wird eine Lösung aus 43,1 g Bismutnitrat in 100 ml 50 %iger Salpetersäure zugetropft, wobei durch Zugabe von Ammoniaklösung ein pH-Wert zwischen 7 und 8 eingestellt wird. Die Mischung wird eingedampft und der getrocknete Katalysatorvorläufer bei 600°C für 12 Stunden calciniert. Der Katalysator wird nach dem Abkühlen auf Raumtemperatur im Mörser homogenisiert und das Pulver zu einer Siebfraktion (18 bis 25 mesh) verarbeitet.

Die Oxidation wird wie in Beispiel 5 beschrieben durchgeführt. Die Ergebnisse sind in Tabelle 1 angegeben.

### Vergleichsbeispiel 2

4 g Oxovanadium(lV)-diphosphat wird nach den Angaben gemäß DD-PS 113 210 präpariert und als Siebfraktion in den Reaktor eingefüllt. Entsprechend DD 298 234, Beispiel 1, wird das Eduktgas bestehend aus 1,5 Vol.-% 4-Chlortoluol, 54 Vol.-% Luft und 44,5 Vol.-% Wasserdampf (Molverhältnis 4-Chlortoluol:Sauerstoff:Wasser = 1:7:30) mit einer Verweilzeit von 1,0 s durch den Reaktor geleitet. Die Reaktionstemperatur beträgt 425°C. Zur Analyse des Produktstroms wird wie in Beispiel 1 beschrieben verfahren. Bei einem Umsatz von 26 % beträgt die 4-Chlorbenzaldehydselektivität 27 %. Die Ausbeute beträgt 7 %.

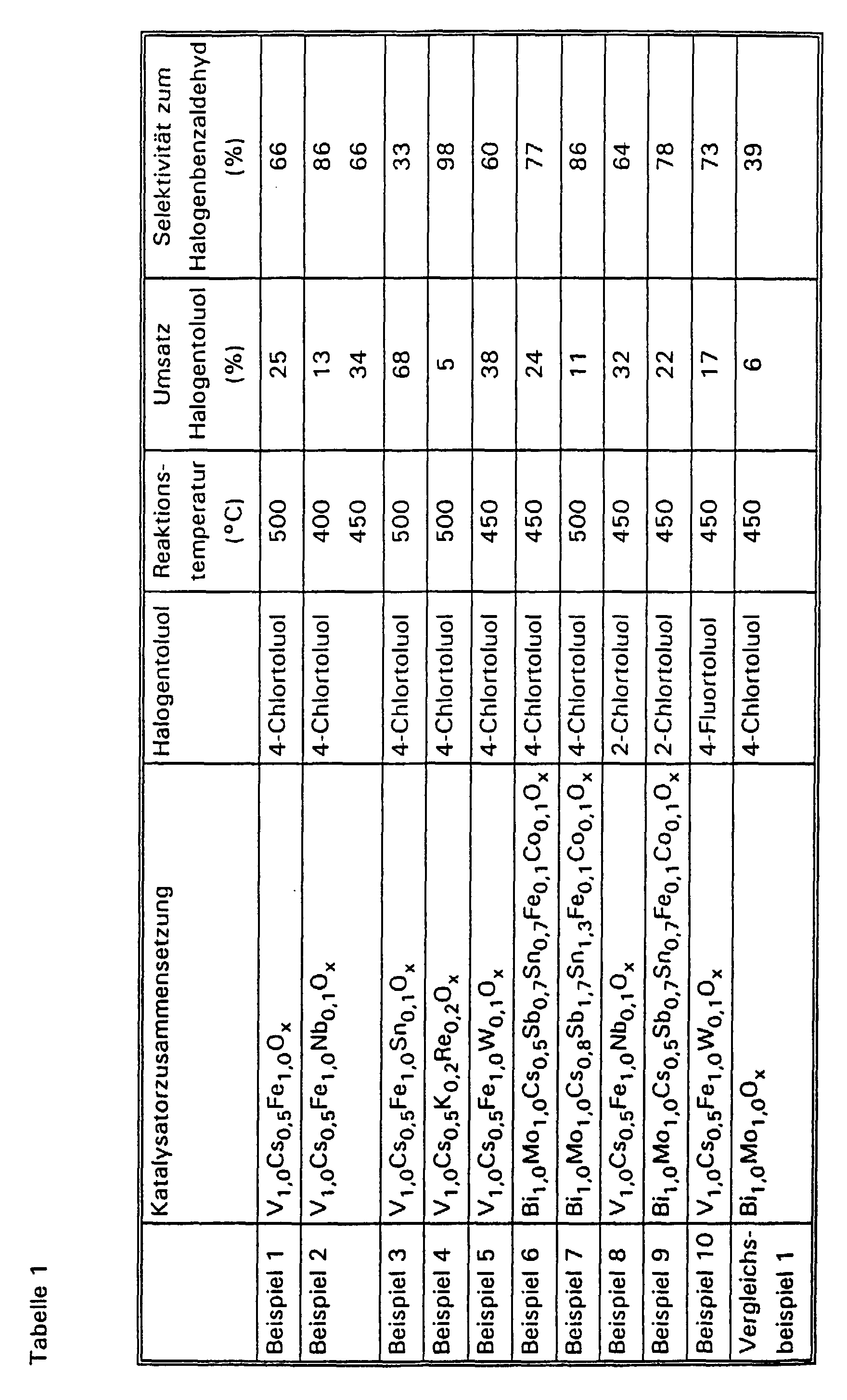

## Patentansprüche

1. Verfahren zur Herstellung von Halogenbenzaldehyden der allgemeinen Formel (I): 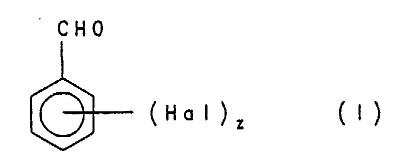 in welcher Hal für Fluor, Chlor, Brom oder Jod steht und z = 1, 2, 3 oder 4 ist, durch katalytische Gasphasenoxidation eines substituierten Toluols der allgemeinen Formel (II), 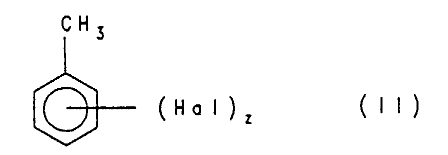 mit Sauerstoff, dadurch gekennzeichnet, daß die Oxidation in Gegenwart eines Katalysators der allgemeinen Formel (III)
Me¹ ₐMe² _{b}Me³ _{c}Oₓ (III)
durchgeführt wird, in der Me¹ für Wismut oder Vanadium, Me² für mindestens eines der Elemente aus der Gruppe bestehend aus Lithium, Natrium, Kalium, Rubidium und Cäsium, Me³ für mindestens ein Element aus der Gruppe bestehend aus Eisen, Kobalt, Nickel, Niob, Molybdän, Arsen, Zinn, Antimon, Wolfram, Tantal, Phosphor, Chrom, Mangan, Palladium, Kupfer, Zink, Cer, Silber, Bor, Samarium, Barium, Calcium, Magnesium und Rhenium stehen, und die Buchstaben a, b und c ein solches Atomverhältnis der jeweiligen Elemente darstellen, daß dann wenn a = 1 angenommen wird, b einen Wert im Bereich von 0,1 bis 2 und c einen Wert im Bereich von 0,05 bis 2 hat, wobei für unterschiedliche Elemente Me² oder Me³ unterschiedliche Werte für b bzw. c möglich sind.

2. Verfahren nach Anspruch 1, dadurch gekennzeichnet, daß Me³ für mindestens ein Element aus der Gruppe bestehend aus Eisen, Kobalt, Nickel, Niob, Molybdän, Arsen, Zinn, Antimon und Rhenium steht.

3. Verfahren nach Anspruch 1, dadurch gekennzeichnet, daß Me¹ Vanadium, Me² Cäsium und Me³ mindestens ein Element aus der Gruppe bestehend aus Eisen, Niob, Arsen, Zinn, Antimon, Rhenium und Wolfram ist, insbesondere die Kombination V₁Cs_{0,5-1}Fe_{0,5-2}(Nb, Sb, Sn, Re oder W)_{0,05-0,2}Oₓ.

4. Verfahren nach Anspruch 1, dadurch gekennzeichnet, daß Me¹ Wismut, Me² Cäsium und Me³ mindestens ein Element aus der Gruppe bestehend aus Molybdän, Zinn, Antimon, Eisen und Kobalt ist, insbesondere die Kombination Bi₁Cs_{0,5-2}(Sb und/oder Sn)_{0,2-1,5}Oₓ.

5. Verfahren nach mindestens einem der Ansprüche 1 bis 4, dadurch gekennzeichnet, daß der Katalysator in reiner Form vorliegt, mit einem Trägermaterial gemischt ist oder auf einem geformten Trägermaterial fixiert ist.

6. Verfahren nach Anspruch 2, dadurch gekennzeichnet, daß der Katalysator auf einem Trägermaterial fixiert eingesetzt wird.

7. Verfahren nach mindestens einem der Ansprüche 1 bis 5, dadurch gekennzeichnet, daß als Trägermaterial Aluminiumoxid, Keramik, Kieselgur, Kieselgel, Siliciumcarbid, geglühtes Siliciumdioxid oder Titandioxid verwendet wird.

8. Verfahren nach Anspruch 3, dadurch gekennzeichnet, daß der Katalysator in reiner Form eingesetzt wird.

9. Verfahren nach mindestens einem der Ansprüche 1 bis 8, dadurch gekennzeichnet, daß die Oxidation in Wirbelschicht oder einem Festbettreaktor ausgeführt wird.

10. Verfahren nach mindestens einem der Ansprüche 1 bis 9, dadurch gekennzeichnet, daß als Oxidationsmittel ein Gemisch aus Sauerstoff mit einem inerten Gas, insbesondere Stickstoff, Kohlendioxid, Argon, bevorzugt Luft eingesetzt wird.

11. Verfahren nach mindestens einem der Ansprüche 1 bis 10, dadurch gekennzeichnet, daß die Reaktionstemperatur 400 bis 500°C beträgt.

12. Verfahren nach mindestens einem der Ansprüche 1 bis 11, dadurch gekennzeichnet, daß in Formel (I) und (II) Hal für Fluor, Chlor oder Brom, insbesondere Chlor oder Brom, bevorzugt Chlor steht.

13. Verfahren nach mindestens einem der Ansprüche 1 bis 12, dadurch gekennzeichnet, daß in Formel (I) und (II) z für 1 oder 2, insbesondere 1 steht.

14. Verfahren nach mindestens einem der Ansprüche 1 bis 13, dadurch gekennzeichnet, daß 2-Chlortoluol, 4-Chlortoluol, 4-Fluortoluol oder 2-Bromtoluol eingesetzt werden.

## Claims

1. A process for the preparation of halobenzaldehydes of the formula (I): 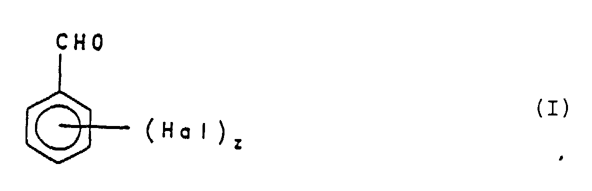 in which Hal is fluorine, chlorine, bromine or iodine and z = 1, 2, 3 or 4, by catalytic gas-phase oxidation of a substituted toluene of the formula (II), 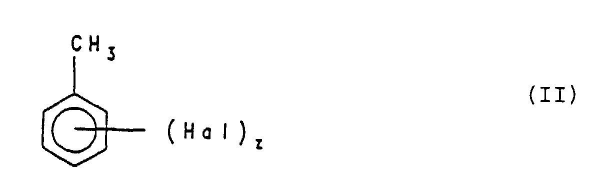 by oxygen, which comprises carrying out the oxidation in the presence of a catalyst of the formula (III)
Me¹ ₐMe² _{b}Me³ _{c}Oₓ (III),
in which Me¹ is bismuth or vanadium, Me² is at least one of the elements selected from the group consisting of lithium, sodium, potassium, rubidium and cesium, Me³ is at least one element selected from the group consisting of iron, cobalt, nickel, niobium, molybdenum, arsenic, tin, antimony, tungsten, tantalum, phosphorus, chromium, manganese, palladium, copper, zinc, cerium, silver, boron, samarium, barium, calcium, magnesium and rhenium, and the letters a, b and c represent an atomic ratio of the respective elements such that when a = 1, b has a value in the range from 0.1 to 2 and c has a value in the range from 0.05 to 2, different values for b and c being possible for different elements Me² or Me³.

2. The process as claimed in claim 1, wherein Me³ is at least one element selected from the group consisting of iron, cobalt, nickel, niobium, molybdenum, arsenic, tin, antimony and rhenium.

3. The process as claimed in claim 1, wherein Me¹ is vanadium, Me² is cesium and Me³ is at least one element selected from the group consisting of iron, niobium, arsenic, tin, antimony, rhenium and tungsten, in particular the combination V₁Cs_{0.5-1}Fe_{0.5-2}(Nb, Sb, Sn, Re or W)_{0.05-0.2}Oₓ.

4. The process as claimed in claim 1, wherein Me¹ is bismuth, Me² is cesium and Me³ is at least one element selected from the group consisting of molybdenum, tin, antimony, iron and cobalt, in particular the combination Bi₁Cs_{0.5-2}(Sb and/or Sn)_{0.2-1.5}Oₓ.

5. The process as claimed in at least one of claims 1 to 4, wherein the catalyst is present in pure form, is mixed with a support material or is fixed on a molded support material.

6. The process as claimed in claim 2, wherein the catalyst is used fixed on a support material.

7. The process as claimed in at least one of claims 1 to 5, wherein the support material used is alumina, ceramic, kieselguhr, silica gel, silicon carbide, fused silica or titanium dioxide.

8. The process as claimed in claim 3, wherein the catalyst is used in pure form.

9. The process as claimed in at least one of claims 1 to 8, wherein the oxidation is carried out in a fluidized bed or a fixed-bed reactor.

10. The process as claimed in at least one of claims 1 to 9, wherein the oxidizing agent used is a mixture of oxygen with an inert gas, in particular nitrogen, carbon dioxide, argon, preferably air.

11. The process as claimed in at least one of claims 1 to 10, wherein the reaction temperature is 400 to 500°C.

12. The process as claimed in at least one of claims 1 to 11, wherein Hal is fluorine, chlorine or bromine, in particular chlorine or bromine, preferably chlorine, in formulae (I) and (II).

13. The process as claimed in at least one of claims 1 to 12, wherein z is 1 or 2, in particular 1, in formulae (I) and (II).

14. The process as claimed in at least one of claims 1 to 13, wherein 2-chlorotoluene, 4-chlorotoluene, 4-fluorotoluene or 2-bromotoluene is used.

## Revendications

1. Procédé de préparation de benzaldéhydes halogénés de formule générale (I) : 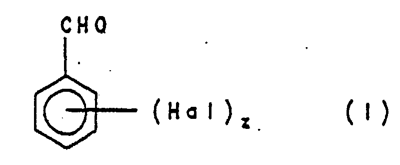 dans laquelle Hal représente le fluor, le chlore, le brome ou l'iode et z = 1, 2, 3 ou 4, par oxydation catalytique en phase gazeuse d'un toluène substitué de formule générale (II), 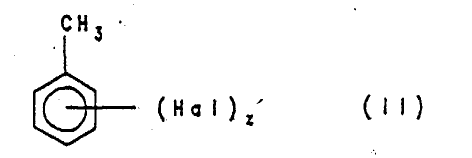 avec de l'oxygène, caractérisé en ce que l'oxydation est réalisée en présence d'un catalyseur de formule générale (III)
Me¹ ₐMe² _{b}Me³ _{c}Oₓ (III),
dans laquelle **Me**^{**1**} représente le bismuth ou le vanadium, **Me**^{**2**} représente au moins un des éléments choisis parmi le lithium, le sodium, le potassium, le ruthénium et le césium, **Me**^{**3**} représente au moins un élément choisi parmi le fer, le cobalt, le nickel, le niobium, le molybdène, l'arsenic, l'étain, l'antimoine, le tungstène, le tantale, le phosphore, le chrome, le manganèse, le palladium, le cuivre, le zinc, le cérium, l'argent, le bore, le samarium, le baryum, le calcium, le magnésium et le rhénium, et les voyelles a, b et c représentent un rapport atomique des éléments respectifs tel que lorsqu'on prend a = 1, b a une valeur dans la gamme de 0,1 à 2 et c une valeur dans la gamme de 0,05 à 2, dans laquelle différentes valeurs pour b ou c sont possibles pour les différents éléments **Me**^{**2**} ou **Me**^{**3**}.

2. Procédé selon la revendication 1, caractérisé en ce que **Me**^{**3**} représente au moins un élément choisi parmi le fer, le cobalt, le nickel, le niobium, le molybdène, l'arsenic, l'étain, l'antimoine et le rhénium.

3. Procédé selon la revendication 1, caractérisé en ce que **Me**^{**1**} est un atome de vanadium, **Me**^{**2**} est un atome de césium et **Me**^{**3**} est au moins un élément choisi parmi le fer, le niobium, l'arsenic, l'étain, l'antimoine, .le rhénium et le tungstène, en particulier la combinaison V₁Cs_{0,5-1}Fe_{0,5-2}(Nb, Sb, Sn, Re ou W)_{0,05-0,2}Oₓ.

4. Procédé selon la revendication 1, caractérisé en ce que **Me**^{**1**} est un atome de bismuth, **Me**^{**2**} est un atome de césium et **Me**^{**3**} est au moins un élément choisi parmi le molybdène, l'étain, l'antimoine, le fer et le cobalt, en particulier la combinaison Bi₁Cs_{0,5-2}(Sb et/ou Sn)_{0,2-1,5}Oₓ.

5. Procédé selon au moins une des revendications 1 à 4, caractérisé en ce que, le catalyseur se présente sous forme pure, est mélangé avec un matériau support ou est fixé sur un matériau support mis en forme.

6. Procédé selon la revendication 2, caractérisé en ce que, le catalyseur est utilisé fixé sur un matériau support.

7. Procédé selon au moins une des revendications 1 à 5, caractérisé en ce qu'on utilise comme matériau support l'oxyde d'aluminium, la céramique, la diatomite, le gel de silice, le carbure de silicium, le dioxyde de silicium recuit ou le dioxyde de titane.

8. Procédé selon la revendication 3, caractérisé en ce que, le catalyseur est utilisé sous forme pure.

9. Procédé selon au moins une des revendications 1 à 8, caractérisé en ce qu'on réalise l'oxydation dans un lit fluidisé ou dans un réacteur à lit fixe.

10. Procédé selon au moins une des revendications 1 à 9, caractérisé en ce qu'on utilise comme agent d'oxydation un mélange d'oxygène avec un gaz inerte, en particulier l'azote, le dioxyde de carbone, l'argon, de préférence l'air.

11. Procédé selon au moins une des revendications 1 à 10, caractérisé en ce que la température de réaction s'élève à 400 à 500°C.

12. Procédé selon au moins une des revendications 1 à 11, caractérisé en ce que dans les formules (I) et (II) Hal représente le fluor, le chlore ou le brome, en particulier le chlore ou le brome, de préférence le chlore.

13. Procédé selon au moins une des revendications 1 à 12, caractérisé en ce que dans les formules (I) et (II) z représente 1 ou 2, en particulier 1.

14. Procédé selon au moins une des revendications 1 à 13, caractérisé en ce qu'on utilise le 2-chlorotoluène, le 4-chlorotoluène, le 4-fluorotoluène ou le 2-bromotoluène.
